# EUROPEAN PATENT APPLICATION

(11) **EP 3 949 729 A1**
(43) Date of publication of application: **09.02.2022**
(21) Application number: 20195540.8
(22) Date of filing: 10.09.2020
(51) Int. Cl.: A01K 13/00

(54) **NOVEL PET COMB WITH ILLUMINATION AND ULTRAVIOLET STERILIZATION LAMP**

(30) Priority: 08.08.2020 CN 202010792126
(71) Applicant: Jiangmen Furong Electrical Products Company Limited, Jiangmen City, Guangdong Province (CN)
(72) Inventor: NG, Ki Churk, Jiangmen City, Guangdong Province (CN)
(74) Representative: Tahtadjiev, Konstantin

(57) **Abstract**

A pet comb with an illumination and ultraviolet sterilization lamp includes a front shell. A bottom shell is provided at an opening of the front shell. A battery cover is provided on a side of the bottom shell away from the front shell. A decorative cover is provided on a side of the front shell away from the bottom shell. A button is provided on the surface of the decorative cover. A circuit board is provided inside the front cover. A sheet ultraviolet light is provided on the comb head base and configured to sterilize hairs of a pet via the ultraviolet rays emitted from the sheet ultraviolet light while grooming the pet, which provides improved sanitization and health for the pet. Moreover, the sheet ultraviolet light can be replaced with an illumination lamp, so that the comb may be used in poorly lighted or dark environments.

## Description

### TECHNICAL FIELD

The present invention relates to the technical field of pet products, and more particularly, to a novel pet comb with an illumination and ultraviolet sterilization lamp.

### BACKGROUND

At present, pet combs available on the market do not have a sterilizing function when grooming pets. Moreover, the head of the pet comb cannot be replaced with various cutter heads and thus cannot fit different pets, which narrows the application range. Additionally, prior pet combs do not have the illumination function and thus cannot be used to groom pets in poorly lit or dark environments. It is, therefore, highly desirable to develop a novel pet comb with an illumination and ultraviolet sterilization lamp.

### SUMMARY

An objective of the present invention is to provide a novel pet comb with an illumination and ultraviolet sterilization lamp to solve the above-mentioned shortcomings of the prior art.

To achieve the above-mentioned objective, the present invention provides the following technical solution. A novel pet comb with an illumination and ultraviolet sterilization lamp includes a front shell. A bottom shell is provided at an opening of the front shell. A battery cover is provided on one side of the bottom shell away from the front shell. A decorative cover is provided on one side of the front shell away from the bottom shell. A button is provided on the surface of the decorative cover. A circuit board is provided inside the front shell. A dry battery is provided inside the bottom shell and arranged between the battery cover and the bottom shell. One side of the inside of the bottom shell adjacent to a negative electrode of the dry battery is provided with a conductive sheet positive electrode. One side of the inside of the bottom shell adjacent to a positive electrode of the dry battery is provided with a conductive sheet negative electrode. A conductive sheet with positive and negative electrodes is provided on the circuit board. One side of the front shell away from the bottom shell is provided with a comb head base at the opening of the front shell. Comb teeth are provided in the comb head base. A sheet ultraviolet light is installed in the comb head base.

Preferably, a threaded column is installed inside the front shell. A bottom shell screw is provided on the surface of the bottom shell. One end of the bottom shell screw adjacent to the front shell successively passes through the bottom shell and the threaded column and extends to the inside of the threaded column.

Preferably, a battery slot is provided on a side of the bottom shell away from the front shell. The battery cover is buckled to the battery slot. The dry battery is provided inside the battery slot.

Preferably, the surface of the battery cover is provided with an anti-slip protrusion.

Preferably, the conductive sheet positive electrode and the conductive sheet negative electrode are electrically connected to the conductive sheet with positive and negative electrodes on the circuit board through a wire, respectively.

Preferably, the conductive sheet positive electrode is in contact with the negative electrode of the dry battery, and the conductive sheet negative electrode is in contact with the positive electrode of the dry battery.

Preferably, the surface of the front shell is provided with a lanyard.

Preferably, the comb head base is fixedly connected to the front shell by a comb head screw.

Preferably, the button is electrically connected to the circuit board through a wire.

Preferably, the sheet ultraviolet light is electrically connected to the circuit board through a wire.

Compared with the prior art, the present invention has the following advantages:
In the present invention, A sheet ultraviolet light is provided on the comb head base and configured to sterilize hairs of a pet via the ultraviolet rays emitted from the sheet ultraviolet light while grooming the pet, which provides improved sanitization and health for the pet. Moreover, the comb teeth are securely installed in the comb head base by means of buckling, which is easy to disassemble and assemble. In this way, the cutter head of the pet comb can be replaced with various cutter heads to fit different pets, which expands the application range of the product. Additionally, the sheet ultraviolet light can be removed from the comb head base and replaced with an illumination lamp, so that the comb may be used in poorly lighted or dark environments.

### BRIEF DESCRIPTION OF THE DRAWINGS

FIG. 1 is an exploded view showing the structures of the present invention;
FIG. 2 is a top view of the present invention; and
FIG. 3 is a bottom view of the present invention.

In the figures: 1, front shell, 2, bottom shell, 3, battery cover, 4, decorative cover, 5, bottom shell screw, 6, button, 7, circuit board, 8, dry battery, 9, conductive sheet positive electrode, 10, conductive sheet negative electrode, 11, conductive sheet with positive and negative electrodes, 12, lanyard, 13, sheet ultraviolet light, 14, comb head screw, 15, comb head base, 16, comb teeth.

### DETAILED DESCRIPTION OF THE EMBODIMENTS

The technical solutions in the embodiments of the present invention will be described clearly and completely with reference to the drawings of the embodiments of the present invention. Obviously, the described embodiments are only part of the embodiments of the present invention rather than all the embodiments. Based on the embodiments of the present invention, all other embodiments obtained by those skilled in the art without creative efforts shall fall within the scope of protection of the present invention.

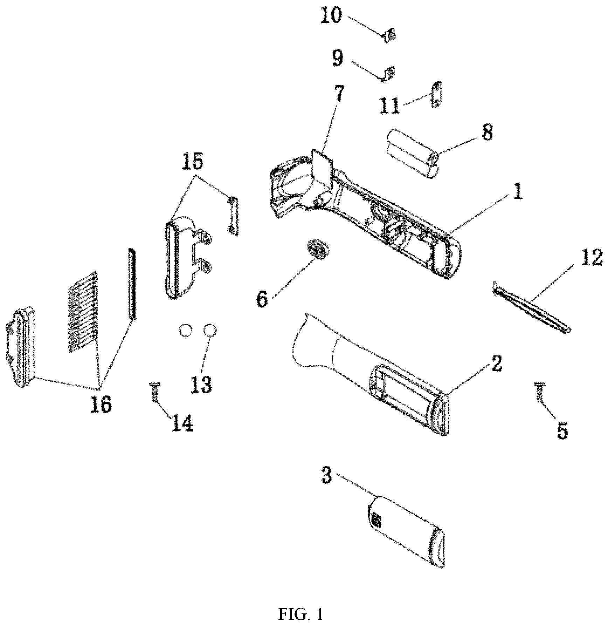

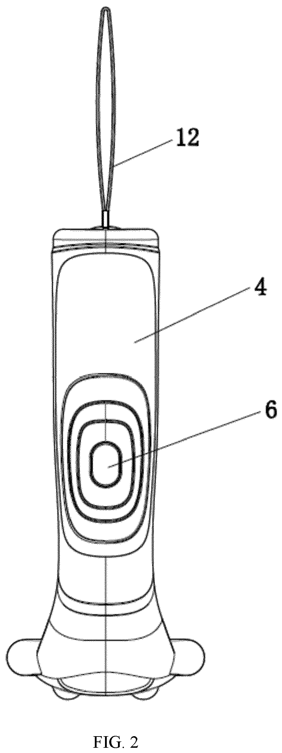

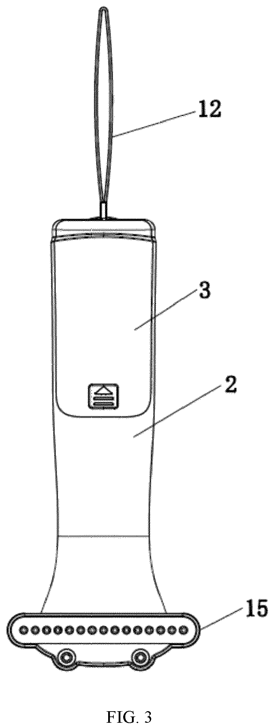

Referring to FIGS. 1-3, a novel pet comb with an illumination and ultraviolet sterilization lamp includes the front shell 1. The bottom shell 2 is provided at the opening of the front shell 1. The battery cover 3 is provided on a side of the bottom shell 2 away from the front shell 1. The decorative cover 4 is provided on a side of the front shell 1 away from the bottom shell 2. The button 6 is provided on the surface of the decorative cover 4. The circuit board 7 is provided inside the front shell 1. The dry battery 8 is provided inside the bottom shell 2 and arranged between the battery cover 3 and the bottom shell 2. One side of the inside of the bottom shell 2 adjacent to the negative electrode of the dry battery 8 is provided with the conductive sheet positive electrode 9. One side of the inside of the bottom shell 2 adjacent to the positive electrode of the dry battery 8 is provided with the conductive sheet negative electrode 10. The conductive sheet 11 with positive and negative electrodes is provided on the circuit board 7. One side of the front shell 1 away from the bottom shell 2 is provided with the comb head base 15 at the opening of the front shell 1. The comb teeth 16 are provided in the comb head base 15. The comb teeth 16 are secured in the comb head base 15 by means of buckling. The sheet ultraviolet light 13 is installed in the comb head base 15.

Specifically, a threaded column is installed inside the front shell 1. The bottom shell screw 5 is provided on the surface of the bottom shell 2. One end of the bottom shell screw 5 adjacent to the front shell 1 successively passes through the bottom shell 2 and the threaded column and extends to the inside of the threaded column.

Specifically, a battery slot is provided on a side of the bottom shell 2 away from the front shell 1. The battery cover 3 is buckled to the battery slot. The dry battery 8 is provided inside the battery slot.

Specifically, the surface of the battery cover 3 is provided with an anti-slip protrusion configured to facilitate opening the battery cover 3.

Specifically, the conductive sheet positive electrode 9 and the conductive sheet negative electrode 10 are electrically connected to the conductive sheet 11 with positive and negative electrodes on the circuit board 7 through a wire, respectively.

Specifically, the conductive sheet positive electrode 9 is in contact with the negative electrode of the dry battery 8, and the conductive sheet negative electrode 10 is in contact with the positive electrode of the dry battery 8.

Specifically, a lanyard 12 is provided on the surface of the front shell 1 so that the comb may be conveniently carried.

Specifically, the comb head base 15 is fixedly connected to the front shell 1 by the comb head screw 14.

Specifically, the button 6 is electrically connected to the circuit board 7 through a wire.

Specifically, the sheet ultraviolet light 13 is electrically connected to the circuit board 7 through a wire, and the sheet ultraviolet light 13 is detachably installed in the comb head base 15.

In use, the button is pressed to turn on the sheet ultraviolet light 13 provided on the comb head base 15. The front shell 1 is held by hand to groom the pet through the comb teeth 16. The ultraviolet rays emitted by the sheet ultraviolet light 13 sterilize the hairs of the pet, which provides improved sanitization and health for the pet. In addition, the comb teeth 16 are installed in the comb base 15 by means of buckling, which is easy to disassemble and assemble. In this way, the cutter head of the pet comb can be replaced with various cutter heads to fit different pets, which extends the range of application for the product. Moreover, the sheet ultraviolet light 13 can be removed from the comb head base 15 and replaced with an illumination lamp so that the pet comb can be used in poorly lit and dark environments.

Although the embodiments of the present invention have been shown and described, those having ordinary skill in the art can understand that various changes, modifications, replacements and variations can be made to these embodiments without departing from the principles and spirit of the present invention, and the scope of the present invention is defined by the appended claims and the equivalents thereof.

## Claims

1. A novel pet comb with an illumination and ultraviolet sterilization lamp, comprising
a front shell (1);
wherein
a bottom shell (2) is provided at an opening of the front shell (1);
a battery cover (3) is provided on a side of the bottom shell (2), wherein the side of the bottom shell is away from the front shell (1);
a decorative cover (4) is provided on a side of the front shell (1), wherein the side of the front shell is away from the bottom shell (2);
a button (6) is provided on a surface of the decorative cover (4); a circuit board (7) is provided inside the front shell (1); a dry battery (8) is provided inside the bottom shell (2) and arranged between the battery cover (3) and the bottom shell (2);
a first side of an inside of the bottom shell (2) is provided with a conductive sheet positive electrode (9), wherein the first side of the inside of the bottom shell (2) is adjacent to a negative electrode of the dry battery (8);
a second side of the inside of the bottom shell (2) is provided with a conductive sheet negative electrode (10), wherein the second side of the inside of the bottom shell (2) is adjacent to a positive electrode of the dry battery (8); and
a conductive sheet (11) with a positive electrode and a negative electrode is provided on the circuit board (7); the side of the front shell (1) away from the bottom shell (2) is provided with a comb head base (15) at the opening of the front shell (1); comb teeth (16) are provided in the comb head base (13); and a sheet ultraviolet light (13) is installed in the comb head base (15).

2. The novel pet comb with the illumination and ultraviolet sterilization lamp according to claim 1, wherein, a threaded column is installed inside the front shell (1); a bottom shell screw (5) is provided on a surface of the bottom shell (2); and an end of the bottom shell screw (5) successively passes through the bottom shell (2) and the threaded column and extends to an inside of the threaded column, wherein the end of the bottom shell (2) is adjacent to the front shell (1).

3. The novel pet comb with the illumination and ultraviolet sterilization lamp according to claim 1, wherein, a battery slot is provided on the side of the bottom shell (2) away from the front shell (1); the battery cover (3) is buckled to the battery slot; and the dry battery (8) is provided inside the battery slot.

4. The novel pet comb with the illumination and ultraviolet sterilization lamp according to claim 1, wherein, a surface of the battery cover (3) is provided with an anti-slip protrusion.

5. The novel pet comb with the illumination and ultraviolet sterilization lamp according to claim 1, wherein, the conductive sheet positive electrode (9) and the conductive sheet negative electrode (10) are electrically connected to the conductive sheet (11) with the positive electrode and the negative electrode on the circuit board (7) through a wire, respectively.

6. The novel pet comb with the illumination and ultraviolet sterilization lamp according to claim 1, wherein, the conductive sheet positive electrode (9) is in contact with the negative electrode of the dry battery (8); and the conductive sheet negative electrode (10) is in contact with the positive electrode of the dry battery (8).

7. The novel pet comb with the illumination and ultraviolet sterilization lamp according to claim 1, wherein, a surface of the front shell (1) is provided with a lanyard (12).

8. The novel pet comb with the illumination and ultraviolet sterilization lamp according to claim 1, wherein, the comb head base (15) is fixedly connected to the front shell (1) by a comb head screw (14).

9. The novel pet comb with the illumination and ultraviolet sterilization lamp according to claim 1, wherein, the button (6) is electrically connected to the circuit board (7) through a wire.

10. The novel pet comb with the illumination and ultraviolet sterilization lamp according to claim 1, wherein, the sheet ultraviolet light (13) is electrically connected to the circuit board (7) through a wire, and the sheet ultraviolet light (13) is detachably installed in the comb head base (15).
